# EUROPEAN PATENT SPECIFICATION

(11) **EP 2 292 218 B1**
(45) Date of publication and mention of the grant of the patent: **11.11.2015**
(21) Application number: 10174934.9
(22) Date of filing: 01.09.2010
(51) Int. Cl.: A61K 9/70, A61K 31/18, A61K 31/704

(54) **Topical application of nimesulide and thiocolchicoside**
Nimesulid und thiocolchicosid enthaltende topische formulierungen
Formulation topique comprenant du nimesulide et du thiocolchicoside

(30) Priority: 02.09.2009 TR 200906775
(43) Date of publication of application: 09.03.2011
(73) Proprietor: Sanovel Ilaç Sanayi Ve Ticaret Anonim Sirketi, 34460 Sariyer/Istanbul (TR)
(72) Inventor: Cakir, Fatih, 34398, Istanbul (TR); Turkyilmaz, Ali, 34398, Istanbul (TR); Cifter, Ümit, 34398, Istanbul (TR); Oner, Levent, 34398, Istanbul (TR)
(74) Representative: Sevinç, Erkan

(56) References cited:
- EP-A1- 0 782 855
- WO-A2-98/01124
- WO-A2-2007/129162

## Description

### Field of Invention

The present invention relates to novel pharmaceutical combinations of nimesulide, and thiocolchicoside, with anti-inflammatory, analgesic, and myorelaxant activities.

The present invention more particularly relates to pharmaceutical combinations of nimesulide and thiocolchicoside, in the form of topically-administered gel, ointment, cream, spray, or lotion with anti-inflammatory, analgesic, and myorelaxant activities. The rate of percutaneous penetration of said combination is enhanced with the auxiliaries it contains.

### Background of Invention

Nimesulide is a non-steroidal anti-inflammatory (NSAID) molecule with the chemical structure illustrated in the following Formula 1.

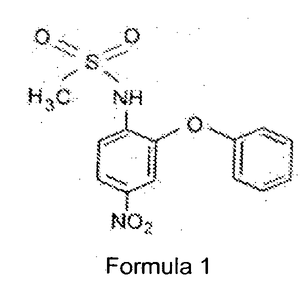

Nimesulide selectively inhibits the cyclooxygenase-2 (COX-2) enzyme. It has antipyretic, analgesic, and anti-inflammatory effects. It is used in osteoarthritis and extraarticular rheumatic diseases, post-traumatic and post-operative inflammations and painful symptoms, high fever, and in dysmenorrhea.

Thiocolchicoside is a myorelaxant, with the following structure illustrated in Formula 2.

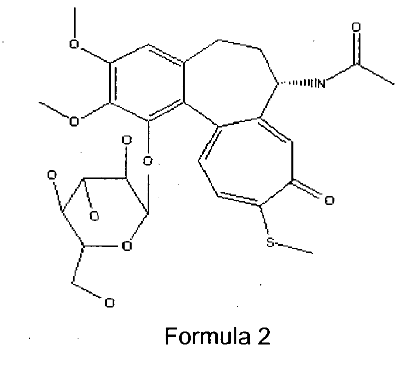

Thiocolchicoside is a semi-synthetic sulfur derivative, obtained from colchicoside, a natural glycoside found in Anatolian Colchicum Autumnale (*Autumn crocuses*). This natural glycoside muscle relaxant has anti-inflammatory, analgesic effects. Thiocolchicoside exerts the myorelaxant effect by activating GABA and glycine receptors at the spinal level.

Glycinomimetic effects of thiocolchicoside are seen on the nerve system at varying levels. It does not have curarizing effect. It does not cause to motor plaque paralysis, and no inhalation-related problems are encountered. It does not have effects on the cardiovascular system either.

Muscle relaxants also reduce the muscle tonus and are used in treating muscle spasms and contractures. Muscle spasm is one of the main factors held responsible for chronic pains; in addition to rheumatic inflammatory and degenerative orthopedic pathologies, it defines various pathologies of the locomotor system as well; when it effects the joints, it does not cause pain only, but it leads to stiffness that reduces the mobility and flexibility of joints at the affected site.

Muscle contractures also characterizes various pathologies of the locomotor system, and is one of the main causes, which is deemed responsible for the persistency of pain associated with such pathologies.

Muscle relaxants are used in neuromuscular and muscle-skeleton system damages. There are two main types of muscle relaxants: centrally-acting muscle relaxants and directly-acting muscle relaxants.

Centrally-acting muscle relaxants typically act on the central nervous system (CNS) in a selective manner and are primarily used for alleviating painful muscle spasms and the strains occurring during muscle-skeleton system and neuromuscular damages. The action mechanisms thereof are associated with the causes of CNS-suppressing activities.

Accordingly, muscle relaxants and antispasmodic molecules constitute a subject matter which is still clinically significant.

Recently, it has been reported that the activity of thiocolchicoside was based on its capability of interacting with strychnine-sensitive glycine receptors, and therefore compounds with glycinomimetic activities have been introduced for use in rheumatologic-orthopedic fields as muscle relaxants.

Muscle relaxants are used alone or together with customary analgesics in treating pain. In fact, a pharmaceutical combination may give complex or unforeseeable outcomes; but so far, nimesulide has never been used together with thiocolchicoside in a pharmaceutical combination for treating inflammatory-, pain, and muscle-skeletal system diseases.

Researching the patent literature may result in various patents, which relates to nimesulide and thiocolchicoside.

For example the patent EP0782855B1 (Helsinn Healthcare SA) discloses an externally-used anti-inflammatory agent, containing nimesulide as the active ingredient, together with a base. There, a pharmaceutical preparation is obtained by mixing 0.1 to 5% by weight of nimesulide in a gel cream base comprising 0.2 to 3% by weight of a hydrophilic polymer, 2 to 20% by weight of an oily substance, 0.5 to 7% by weight of a nonionic surface active agent, 0.01 to 5% by weight of a basic substance, and 50 to 90% by weight of water.

WO 2007/129162 A2 discloses formulations for transdermal use comprising colchicine and a compound having pharmacological activity such as ibuprofen. It is reported that penetration through subcutaneous level is improved by the alleged synergy between colchicine and emulgel.

The patent EP0853476B1 (Errekappa Euroterapici SpA) discloses a pharmaceutical preparation containing as active principle nimesulide or one of its active derivatives, **characterized in that** the preparation's base contains one phospholipid and at least one substance with acid reaction, specifically an acid.

The patent FR 2 725 134 B1 discloses a novel pharmaceutical combination containing ibuprofen or a pharmaceutically acceptable salt thereof and thiocolchicoside or a pharmaceutically acceptable salt thereof in a proportion ranging from 1:50 to 1:200.

According to that invention, said pharmaceutical combination is useful in treating painful muscle syndromes and more specifically in treating lumbagos.

The patent EP 0 837 684 B1 discloses pharmaceutical compositions containing, in solid form, a diclofenac salt and thiocolchicoside, combined with at least one pharmaceutically acceptable carrier, for use in therapy. According to that invention, the muscle relaxant drug used in combination with flurbiprofen is metocarbamol and this combination is particularly used in treating influenza and common cold symptoms involving sore throat.

There has been no pharmaceutical compositions or dosage forms produced until today, which contain the combination of thiocolchicoside and nimesulide. Even if muscle relaxants and non-steroidal inflammatory drugs have been used together in practice, this fact requires the patients to carry more than one drugs and causes application-related difficulties.

One of the drawbacks due to nimesulide, as one of the active ingredients used, is the resulting lower percutaneous penetration, requiring relatively longer time periods following the administration thereof. Particularly in acute disorders, there arises the need of enhancing the absorption rate at the site of administration. Even if there is not any problem associated with the absorption of thiocolchicoside, which is the other active ingredient used in the formulation, the requirement is obvious to increase the rate of absorption particularly during acute situations.

Whilst it is known that surface-active agents and excipients providing percutaneous penetration are used for eliminating the aforesaid problem, particularly the percutaneous penetration enhancers bring about irritations at the primary site of administration.

Commercially-available products containing thiocolchicoside form yellow spots at the administration site. Commercially-available nimesulide containing products similarly form skin stains. These facts are because of the structural features of both active ingredients. Both substances are yellow and the products in which these active agents are present cause aesthetically-undesired color formations following their administration.

In result, the aforesaid drawbacks require a novelty in the art of pharmaceutical combinations with anti-inflammatory, analgesic, and myorelaxant activities.

### Object and Brief Description of Invention

The present invention relates to an easily applicable nimesulide and thiocolchicoside and method combination, eliminating all aforesaid problems and brining additional advantages to the relevant prior art.

Accordingly, the main object of the present invention is to obtain a stable combination of nimesulide and thiocolchicoside and method with anti-inflammatory, analgesic, and myorelaxant activities.

Another object of the present invention is to enhance the percutaneous penetration rate of the subject combination by additional excipients and meanwhile to avoid irritation of the skin.

A further object of the present invention is to improve the subject's psychological comfort with the freshening effect of menthol, which when used in said combination further enhances the percutaneous penetration thereof.

Yet a further object of the present invention is to obtain a product, allowing to keep the possible stain problem to occur at the site of administration at an acceptable level.

Accordingly, a topical pharmaceutical formulation has been developed to achieve all objects referred to above and to emerge from the following detailed description.

In a preferred embodiment according to the present invention, said novelty is realized with nimesulide together with thiocolchicoside and method.

The amount of menthol makes up 0.05 to 10%, preferably 0.1 to 5%, and more preferably 0.25 to 3% of the total weight of composition.

In a further preferred embodiment according to the present invention, polysorbate is used as a surface active agent.

In another preferred embodiment according to the present invention, at least one or a mixture of dimethyl sulfoxide, ethyl alcohol, and/or polysorbate is used as a percutaneous penetration enhancer. The amount of said percutaneous penetration enhancer makes up 0.05 to 5%, preferably 0.1 to 4%, and more preferably 0.25 to 3% of the total weight of composition.

In a further preferred embodiment according to the present invention, at least one or a mixture of carbomer and/or triethanolamine is used as a viscosity and gellifying enhancer.

In another preferred embodiment according to the present invention, polyethylene glycol is used as a dissolving agent.

In a further preferred embodiment according to the present invention, disodium EDTA is used as a chelating agent.

In another preferred embodiment according to the present invention, glycerin is used as a viscosity enhancer.

In a further preferred embodiment according to the present invention, the amount of nimesulide makes up 0.10 to 5%, preferably 0.10 to 4%, and more preferably 0.25 to 4% of the total weight of composition, whereas the amount of thiocolchicoside makes up 0.05 to 7%, preferably 0.10 to 6%, and more preferably 0.10 to 5% of the total weight of composition.

Another aspect of the present invention provides a method for preparing the pharmaceutical formulation according to the present invention, this method comprising the steps of
a. adding disodium EDTA into water, then adding carbomer therein and stirring the resultant mixture so as to swell the later and obtain the first mixture;
b. adding and dissolving nimesulide into polyethylene glycol in a separate container, then adding and dissolving thiocolchicoside into this mixture, then adding dimethyl sulfoxide into and stirring it in the resultant mixture so as to obtain the second mixture;
c. adding glycerin and menthol previously dissolved in alcohol into the second mixture, then adding polysorbate therein and stirring the resultant mixture;
d. adding the second mixture into the first mixture under stirring; and
e. adding triethanolamine into the resultant final mixture, then gellifying and stirring this mixture with adding water into it.

In a further preferred embodiment of the present invention, said pharmaceutical formulation consisting of

| | |
|---|---|
| a. nimesulide | at 0.10 to 5% by weight |
| b. thiocolchicoside | at 0.10 to 5% by weight |
| c. menthol | at 0.05 to 10% by weight |
| d. disodium EDTA | at 0.002 to 0.30% by weight |
| e. carbomer | at 0.10 to 4% by weight |
| f. polyethylene glycol | at 2 to 50% by weight |
| g. triethanolamine | at 0.10 to 5% by weight |
| h. dimethyl sulfoxide | at 2 to 50% by weight |
| i. glycerin | at 10 to 50% by weight |
| j. polysorbate | at 0.10 to 15% by weight |
| k. ethyl alcohol | at 2 to 50% by weight |
| l. purified water | at 30 to 60% by weight. |

### Detailed Description of Invention

### Example:

| Content | amount (%) (w/w) |
|---|---|
| nimesulide | 1 |
| thiocolchicoside | 0.25 |
| menthol | 2.50 |
| disodium EDTA | 0.10 |
| carbomer 940 | 1 |
| polyethylene glycol 400 | 20 |
| triethanolamine | 0.20 |
| dimethyl sulfoxide | 6 |
| glycerin | 10 |
| polysorbate 80 | 2 |
| ethyl alcohol | 10 |
| purified water | 46.95 |

Disodium EDTA is added into water under stirring, then carbomer 940 is added therein and the resultant mixture is stirred and is made to swell. Thus, the first mixture is obtained. In a separate container, nimesulide is added into polyethylene glycol 400 and dissolved therein.

Then thiocolchicoside is added and dissolved into the resulting mixture of nimesulide, then dimethyl sulfoxide is added therein and the mixture is stirred. Into this mixture formed is added glycerin and then menthol, which is previously dissolved in alcohol. Then polysorbate 80 is added into this mixture and it is stirred, giving the second mixture. The second mixture is added into the first mixture under stirring. Into the resultant final mixture is added triethanolamine, then it is brought into a gellified state and the procedure is completed with the addition of water therein.

Accordingly, a novel formulation with anti-inflammatory, analgesic, and myorelaxant activities is developed, which surprisingly is rapidly absorbed by the skin without irritating the skin and avoiding the staining problem probably to occur thereat.

This formulation also provides desired stability levels. Thus, the formulation's shelf life is brought to a desired level.

The pharmaceutical compositions according to the present invention may also comprise one or more pharmaceutically acceptable auxiliaries. Such proper pharmaceutically acceptable auxiliaries includes, but are not restricted to gel forming agents, viscosity enhancers, surface active agents, penetration enhancers, chelating agents, preservatives, antioxidants, odor masking agents, solvents etc. and mixtures thereof.

Menthol, as used in the formulation according to the present invention, both increases trhe percutaneous penetration rate, and gives the subject mental and psychological relief with the freshening effect it exerts on the site of administration. It also eliminates unwanted odors possibly to occur. The amount of menthol makes up 0.05 to 10%, preferably 0.10 to 5%, and more preferably 0.25 to 3% of the total weight of composition. Percutaneous penetration is important with respect to the local efficiency of a product. The adsorption rate of a product following administration affects directly its time course of action and efficiency. The penetration of the menthol-free formulation was found to be low, whereas the formulation with menthol was found to be surprisingly high. The optimum proportion of menthol for both producing a mental, psychological effect as a result of generating a chill effect at the site of administration, and at the same time, for enhancing the penetration values was found to be as 0,25 to 3%.

Polysorbate 80, used as the surface active agent, as well as one or a mixture of dimethyl sulfoxide, ethyl alcohol, and/or polysorbate, used as the percutaneous penetration enhancer raise up the rate of absorption of the composition, thereby substantially shortening the therapy process. The amount of said percutaneous penetration enhancer makes up 0.05 to 5%, preferably 0.1 to 4%, and more preferably 0.25 to 3% of the total weight of composition.

At least one or a mixture of carbomer and/or triethanolamine is used as the viscosity and gellifying enhancer. With adjusting the amount of triethanolamine, the color of the composition is controlled and probable skin stains to occur are prevented.

Accordingly, the present invention provides topical pharmaceutical combinations, comprising nimesulide, and thiocolchicoside, and method in the form of suspensions, ointments, cream, or gels, having anti-inflammatory, analgesic, and myorelaxant activities.

Proper gel forming agents include, but are not restricted to carbomer 940, carbomer 941, gelatin, carbomer copolymer, aluminum monostearat, dextrin, magnesium aluminum silicate, silicon dioxide, sodium alginate, triethanolamine, polyvinyl alcohol, pectin, methylcellulose, hydroxypropyl cellulose and mixtures thereof. The most preferred gel forming agents are carbomer 940 and triethanolamine.

Convenient surface active agents and percutaneous penetration enhancers include, but are not restricted to ethanol, menthol, dimethyl sulfoxide, diethanolamine, glyceryl monostearat, oleic acid, sodium lauril sulfate, propylene glycol, polyethylene glycol succinate, polysorbate 20, polysorbate 40, polysorbate 60, polysorbate 80, etc. and mixtures thereof. The most preferred among those are one or a mixture of polysorbate 80, ethanol, menthol, and/or dimethyl sulfoxide.

Proper chelating agents include, but are not restricted to one or a mixture of calcium EDTA, disodium EDTA, and/or EDTA. The most preferred one among those is disodium EDTA.

The present invention may be used for treating osteoarthritis, pain associated with tissue trauma emerging following osteoarthritis surgery, psoriatic arthritis, rheumatoid arthritis, myalgia, bone pain, pain, arthralgia, muscle spasms, soft tissue traumas, lumbago, back pain, sciatica and torticollis.

The present invention is hereby disclosed by referring to an exemplary embodiment hereinabove. Whilst this exemplary embodiment does not restrict the object of the present invention, the latter must be assessed under the light of the foregoing detailed description.

## Claims

1. A topical pharmaceutical formulation comprising nimesulide together with thiocolchicoside, the formulation further comprising menthol.

2. The pharmaceutical formulation according to claims 1, wherein the amount of menthol is 0.05 to 10% of the total weight of composition, preferably 0.1 to 5% of the total weight of composition, and more preferably to 0.25 to 3% of the total weight of composition.

3. The pharmaceutical formulation according to any of the preceding claims, this pharmaceutical formulation being in the form of gel, ointment, cream, spray, or lotion.

4. The pharmaceutical formulation according to claims 1 and 3, this pharmaceutical formulation being in the form of gel.

5. The pharmaceutical formulation according to any of the preceding claims, comprising polysorbate as a surface active agent.

6. The pharmaceutical formulation according to any of the preceding claims, comprising at least one or a mixture of dimethyl sulfoxide, ethyl alcohol, and/or polysorbate as a percutaneous penetration enhancer.

7. The pharmaceutical formulation according to claims 1 and 4, wherein the amount of said percutaneous penetration enhancer is 0.05 to 5% of the total weight of composition, preferably 0.1 to 4% of the total weight of composition, and more preferably 0.25 to 3% of the total weight of composition.

8. The pharmaceutical formulation according to any of the preceding claims, comprising at least one or a mixture of carbomer and/or triethanolamine as a viscosity and gellifying enhancer.

9. The pharmaceutical formulation according to any of the preceding claims, comprising polyethylene glycol as a dissolving agent.

10. The pharmaceutical formulation according to any of the preceding claims, comprising disodium EDTA as a chelating agent.

11. The pharmaceutical formulation according to any of the preceding claims, comprising glycerin as a viscosity enhancer.

12. The pharmaceutical formulation according to any of the preceding claims, wherein the amount of nimesulide is 0.10 to 5%, preferably 0.10 to 4%, and more preferably 0.25 to 4% of the total weight of composition, whereas the amount of thiocolchicoside is 0.05 to 7%, preferably 0.10 to 6%, and more preferably 0.10 to 5% of the total weight of composition.

13. A method for preparing a pharmaceutical formulation according to any of the preceding claims, comprising the steps of
a. adding disodium EDTA into water, then adding carbomer therein and stirring the resultant mixture so as to swell the later and to obtain the first mixture;
b. adding and dissolving nimesulide into polyethylene glycol in a separate container, then adding and dissolving thiocolchicoside into this mixture, then adding dimethyl sulfoxide into and stirring it in the resultant mixture so as to obtain the second mixture;
c. adding glycerin and menthol previously dissolved in alcohol into the second mixture, then adding polysorbate into the latter and stirring the resultant mixture;
d. adding the second mixture into the first mixture under stirring; and
e. adding triethanolamine into the resultant final mixture, then gellifying and stirring this mixture with adding water into it.

14. A pharmaceutical formulation according to any of the preceding claims, consisting of
| | | |
|---|---|---|
| a. | nimesulide | at 0.10 to 5% by weight |
| b. | thiocolchicoside | at 0.10 to 3.75% by weight |
| c. | menthol | at 0.05 to 10% by weight |
| d. | disodium EDTA | at 0.002 to 0.30% by weight |
| e. | carbomer | at 0.10 to 4% by weight |
| f. | polyethylene glycol | at 2 to 50% by weight |
| g. | triethanolamine | at 0.10 to 5% by weight |
| h. | dimethyl sulfoxide | at 2 to 50% by weight |
| i. | glycerin | at 10 to 50% by weight |
| j. | polysorbate | at 0.10 to 15% by weight |
| k. | ethyl alcohol | at 2 to 50% by weight |
| l. | purified water | at 30 to 60% by weight. |

15. A pharmaceutical formulation according to any of the preceding claims for use in the prevention or treatment of osteoarthritis, pain associated with tissue trauma emerging after osteoarthritis surgery, psoriatic arthritis, rheumatoid arthritis, myalgia, bone pain, pain, arthralgia, muscle spasms, soft tissue traumas, lumbago, back pain, sciatica, and torticollis in mammalians, particularly in humans.

## Patentansprüche

1. Topische pharmazeutische Formulierung umfassend Nimesulid zusammen mit Thiocolchicosid, wobei die Formulierung des Weiteren Menthol umfasst.

2. Pharmazeutische Formulierung nach Anspruch 1, bei der die Mentholmenge zwischen 0,05 und 10 % des Gesamtgewichts der Formulierung, vorzugsweise zwischen 0,1 und 5% des Gesamtgewichts der Formulierung, und noch bevorzugter zwischen 0,25 und 3% des Gesamtgewichts der Formulierung beträgt.

3. Pharmazeutische Formulierung nach einem der vorhergehenden Ansprüche, wobei diese Formulierung in Form eines Gels, einer Salbe, einer Creme, eines Sprays oder einer Lotion vorliegt.

4. Pharmazeutische Formulierung nach den Ansprüchen 1 und 3, wobei die pharmazeutische Formulierung in Form eines Gels vorliegt.

5. Pharmazeutische Formulierung nach einem der vorhergehenden Ansprüche, umfassend Polysorbat als Tensid.

6. Pharmazeutische Formulierung nach einem der vorhergehenden Ansprüche, umfassend zumindest einen der folgenden Stoffe oder ein Gemisch daraus als perkutaner Penetrationsförderer: Dimethylsulfoxid, Ethylalkohol, und/oder Polysorbat.

7. Pharmazeutische Formulierung nach den Ansprüchen 1 und 4, bei der die Menge des perkutanen Penetrationsförderers zwischen 0,05 und 5% des Gesamtgewichts der Zusammensetzung, vorzugsweise zwischen 0,1 und 4% des Gesamtgewichts der Zusammensetzung, und noch bevorzugter zwischen 0,25 und 3% des Gesamtgewichts der Zusammensetzung beträgt.

8. Pharmazeutische Formulierung nach einem der vorhergehenden Ansprüche, umfassend zumindest Carbomer und/oder Triethanolamin oder ein Gemisch daraus als Viskositäts- und Gelierverbesserer.

9. Pharmazeutische Formulierung nach einem der vorhergehenden Ansprüche, umfassend Polyethylenglycol als Lösungsmittel.

10. Pharmazeutische Formulierung nach einem der vorhergehenden Ansprüche, umfassend Dinatrium-EDTA als Chelatbildner.

11. Pharmazeutische Formulierung nach einem der vorhergehenden Ansprüche, umfassend Glyzerin als Viskositätsverbesserer.

12. Pharmazeutische Formulierung nach einem der vorhergehenden Ansprüche, bei der die Nimesulidmenge zwischen 0,10 und 5%, vorzugsweise zwischen 0,10 und 4%, und noch bevorzugter zwischen 0,25 und 4% des Gesamtgewichts der Zusammensetzung beträgt, wohingegen die Thiocolchicosidmenge zwischen 0,05 und 7%, vorzugsweise zwischen 0,10 und 6%, und noch bevorzugter zwischen 0,10 und 5% des Gesamtgewichts der Zusammensetzung beträgt.

13. Verfahren zur Herstellung einer pharmazeutischen Formulierung nach einem der vorhergehen-den Ansprüche, folgende Schritte umfassend:
a) Zugabe von Dinatrium-EDTA zu Wasser, anschließend Zugabe von Carbomer und Verrühren des sich ergebenden Gemisches, um ein Quellen herbeizuführen und das erste Gemisch zu erhalten;
b) Zugabe und Auflösen von Nimesulid in Polyethylenglycol in einem separaten Behältnis, anschließend Zugabe und Auflösen von Thiocolchicosid in diesem Gemisch, anschließend Zugabe und Einrühren von Dimethylsulfoxid in das sich ergebende Gemisch, um das zweite Gemisch zu erhalten;
c) Zugabe von Glyzerin und zuvor in Alkohol gelöstem Menthol in das zweite Gemisch, anschließend Zugabe von Polysorbat in dieses und Umrühren des erhaltenen Gemisches;
d) Einrühren des zweiten Gemisches in das erste Gemisch; und
e) Zugabe von Triethanolamin in das erhaltene Endgemisch, dann Gelierung und Verrühren dieses Gemisches unter Zugabe von Wasser.

14. Pharmazeutische Formulierung nach einem der vorhergehenden Ansprüche, bestehend aus:
| | |
|---|---|
| a. Nimesulid | zu 0,10 bis 5 Gew.-% |
| b. Thiocolchicosid | zu 0,10 bis 3,75 Gew.-% |
| c. Menthol | zu 0,05 bis 10 Gew.-% |
| d. Dinatrium-EDTA | zu 0,002 bis 0,30 Gew.-% |
| e. Carbomer | zu 0,10 bis 4 Gew.-% |
| f. Polyethylenglycol | zu 2 bis 50 Gew.-% |
| g. Triethanolamin | zu 0,10 bis 5 Gew.-% |
| h. Dimethylsulfoxid | zu 2 bis 50 Gew.-% |
| i. Glyzerin | zu 10 bis 50 Gew.-% |
| j. Polysorbat | zu 0,10 bis 15 Gew.-% |
| k. Ethylalkohol | zu 2 bis 50 Gew.-% |
| l. destilliertes Wasser | zu 30 bis 60 Gew.-% |

15. Pharmazeutische Formulierung nach einem der vorhergehenden Ansprüche zur Verwendung zur Prävention oder Behandlung von Osteoarthritis, Schmerzen im Zusammenhang mit Gewebetrauma infolge von Arthroseoperationen, Psoriasisarthritis, Gelenkrheumatismus, Myalgien, Knochenschmerzen, Schmerzen, Arthralgien, Muskelkrämpfen, Weichteiltraumen, Lumbago, Rückenschmerzen, Ischiasbeschwerden und Torticollis bei Säugern, insbesondere bei Menschen.

## Revendications

1. Formulation pharmaceutique topique comprenant du nimésulide et du thiocolchicoside, la formulation comprenant en outre du menthol.

2. Formulation pharmaceutique selon la revendication 1, dans laquelle la quantité de menthol est de 0,05 à 10 % du poids total de la composition, de préférence de 0,1 à 5 % du poids total de la composition, et plus préférentiellement de 0,25 à 3 % du poids total de la composition.

3. Formulation pharmaceutique selon l'une quelconque des revendications précédentes, cette formulation pharmaceutique se présentant sous la forme de gel, de pommade, de crème, de spray ou de lotion.

4. Formulation pharmaceutique selon les revendications 1 et 3, cette formulation pharmaceutique se présentant sous la forme de gel.

5. Formulation pharmaceutique selon l'une quelconque des revendications précédentes, comprenant du polysorbate en tant qu'agent tensioactif.

6. Formulation pharmaceutique selon l'une quelconque des revendications précédentes, comprenant au moins un ou un mélange de diméthylsulfoxide, alcool éthylique, et/ou polysorbate en tant qu'agent d'amélioration de la pénétration percutanée.

7. Formulation pharmaceutique selon les revendications 1 et 4, dans laquelle la quantité dudit agent d'amélioration de la pénétration percutanée constitue de 0,05 à 5 % du poids total de la composition, de préférence de 0,1 à 4 % du poids total de la composition, et plus préférentiellement de 0,25 à 3 % du poids total de la composition.

8. Formulation pharmaceutique selon l'une quelconque des revendications précédentes, comprenant au moins un ou un mélange de carbomer et/ou triéthanolamine en tant qu'agent d'amélioration de la viscosité et de la gélification.

9. Formulation pharmaceutique selon l'une quelconque des revendications précédentes, comprenant du polyéthylène glycol en tant qu'agent dissolvant.

10. Formulation pharmaceutique selon l'une quelconque des revendications précédentes, comprenant de l'EDTA disodique en tant qu'agent chélateur.

11. Formulation pharmaceutique selon l'une quelconque des revendications précédentes, comprenant de la glycérine en tant qu'agent d'amélioration de la viscosité.

12. Formulation pharmaceutique selon l'une quelconque des revendications précédentes, dans laquelle la quantité de nimésulide constitue de 0,10 à 5 %, de préférence de 0,10 à 4 %, et plus préférentiellement de 0,25 à 4 % du poids total de la composition, alors que la quantité de thiocolchicoside constitue de 0,05 à 7 %, de préférence de 0,10 à 6 %, et plus préférentiellement de 0,10 à 5 % du poids total de la composition.

13. Procédé de préparation d'une formulation pharmaceutique selon l'une quelconque des revendications précédentes, comprenant les étapes d'
a. ajouter de l'EDTA disodique dans de l'eau, puis y ajouter du carbomer et agiter le mélange obtenu de manière à l'épaissir afin d'obtenir le premier mélange ;
b. ajouter et dissoudre du nimésulide dans du polyéthylène glycol dans un récipient séparé, puis ajouter et dissoudre du thiocolchicoside dans ce mélange, ajouter ensuite du diméthylsulfoxide et l'agiter dans le mélange obtenu afin d'obtenir le deuxième mélange ;
c. ajouter de la glycérine et du menthol préalablement dissous dans de l'alcool dans le deuxième mélange, puis ajouter du polysorbate dans ce mélange et agiter le mélange obtenu ;
d. ajouter le deuxième mélange dans le premier mélange sous agitation ; et
e. ajouter du triéthanolamine dans le mélange final obtenu, puis gélifier et agiter ce mélange en y ajoutant de l'eau.

14. Formulation pharmaceutique selon l'une quelconque des revendications précédentes, consistant en
| | | |
|---|---|---|
| a. | nimésulide | de 0,10 à 5 % en poids |
| b. | thiocolchicoside | de 0,10 à 3,75 % en poids |
| c. | menthol | de 0,05 à 10 % en poids |
| d. | EDTA disodique | de 0,002 à 0,30 % en poids |
| e. | carbomer | de 0,10 à 4 % en poids |
| f. | polyéthylène glycol | de 2 à 50 % en poids |
| g. | triéthanolamine | de 0,10 à 5 % en poids |
| h. | diméthylsulfoxide | de 2 à 50 % en poids |
| i. | glycérine | de 10 à 50 % en poids |
| j. | polysorbate | de 0,10 à 15 % en poids |
| k. | alcool éthylique | de 2 à 50 % en poids |
| l. | eau purifiée | de 30 à 60 % en poids |

15. Formulation pharmaceutique selon l'une quelconque des revendications précédentes pour son utilisation utilisation en prévention ou traitement de l'ostéo-arthrite, la douleur associée au traumatisme des tissus survenant après chirurgie de l'ostéo-arthrite, l'arthrite psoriatique, l'arthrite rhumatoïde, la myalgie, les douleurs osseuses, la douleur, l'arthralgie, les spasmes musculaires, les traumatismes des tissus mous, le lumbago, les douleurs dorsales, la sciatique, et le torticolis chez les mammifères, plus particulièrement chez l'homme.
